# EUROPEAN PATENT APPLICATION

(11) **EP 4 152 175 A1**
(43) Date of publication of application: **22.03.2023**
(21) Application number: 21818458.8
(22) Date of filing: 20.05.2021
(51) Int. Cl.: G06F 16/2457

(54) **DATA PROCESSING METHOD AND APPARATUS, COMPUTING DEVICE, AND STORAGE MEDIUM**

(30) Priority: 05.06.2020 CN 202010506698
(71) Applicant: Huawei Technologies Co., Ltd., Longgang District, Shenzhen, Guangdong 518129 (CN)
(72) Inventor: LI, Tsz On, Hong Kong, Pok Fu Lam Road, Central West Hong Kong 999077 (CN); JIANG, Jianyu, Hong Kong, Pok Fu Lam Road, Central West Hong Kong 999077 (CN); QI, Ji, Hong Kong, Pok Fu Lam Road, Central West Hong Kong 999077 (CN); SO, Chi Chiu, Hong Kong, Pok Fu Lam Road, Central West Hong Kong 999077 (CN); CUI, Heming, Hong Kong, Pok Fu Lam Road, Central West Hong Kong 999077 (CN); WANG, Sen, Shenzhen, Guangdong 518129 (CN); WANG, Peng, Shenzhen, Guangdong 518129 (CN); ZHANG, Gong, Shenzhen, Guangdong 518129 (CN)
(74) Representative: Goddar, Heinz J.
(86) International application number: PCT/CN2021/094978
(87) International publication number: WO 2021/244303

(57) **Abstract**

Disclosed are a data processing method and apparatus, a computing device, and a storage medium, which belongs to the field of big data technologies. The method includes: determining, based on a query algorithm, a first query output (601) corresponding to an input data set; determining, based on a second query output of an unsampled data record in the input data set and the query algorithm, a perturbation of each of a target quantity of sampled data records in the input data set to the first query output, to obtain sensitivity (602) corresponding to the input data set, where the unsampled data record and the target quantity of data records constitute the input data set; and adding noise to the first query output based on the sensitivity and outputting a noised first query output (603). This can reduce a sensitivity calculation amount, and improve data processing efficiency.

## Description

This application claims priority to Chinese Patent Application No. 202010506698.X, filed on June 05, 2020 and entitled "DATA PROCESSING METHOD AND APPARATUS, COMPUTING DEVICE FOR DATA PROCESSING, AND STORAGE MEDIUM", which is incorporated herein by reference in its entirety.

### TECHNICAL FIELD

This application relates to the field of big data technologies, and in particular, to a data processing method and apparatus, a computing device for data processing, and a storage medium.

### BACKGROUND

With the development of network technologies and computer technologies, organizations pay more attention to privacy protection for personal data. However, even though these organizations have implemented strict protection for security of a big data processing system and anonymity of data (a data inquirer cannot directly obtain original personal data), the data inquirer can still steal personal data by querying the big data processing system. For example, a data inquirer knows that patient 1 has been hospitalized at hospital A, and the data inquirer also knows that patient 1 was the only patient who was hospitalized at hospital A on January 1, 2019. The data inquirer wants to know if patient 1 has cancer. Then the data inquirer queries a big data processing system of hospital A for a quantity of patients who were hospitalized at hospital A on January 1, 2019 and who had cancer. If a query output is 1, the data inquirer determines that patient 1 has cancer. It can be learned that even though the big data processing system of the hospital is secure and data in the big data processing system is anonymous, the data inquirer can still obtain some personal data. Therefore, to more comprehensively protect privacy of personal data, a differential privacy algorithm emerges. The differential privacy algorithm is a privacy protection method that prevents output of a big data processing system from leaking personal data. A principle of the differential privacy algorithm is as follows: The differential privacy algorithm adds noise to an output of the big data processing system. The noise can both prevent the output of the big data processing system from leaking personal data and minimize impact on precision of the output. A specific process is as follows: A third-party data inquirer submits a query algorithm to a server of a data owner. After determining that an input is an input data set, the server provides a query output corresponding to the query algorithm, and then adds noise to the query output by using a differential privacy module based on sensitivity. Finally, the server returns a noised query output to the data inquirer. The sensitivity is a maximum change to the query output after one data record is added to or deleted from the input data set, namely, the maximum perturbation that a single data record can cause to the query output. It can be learned that when the sensitivity is large, a large amount of noise is added to the query output, which seriously affects precision of the query output. On the contrary, when the sensitivity is small, a small amount of noise is added to the query output, which may fail to protect personal privacy. Therefore, determining of the sensitivity is of crucial importance.

In a related technology, a brute-force analysis algorithm is used to determine the sensitivity. Specific processing is as follows: One data record is deleted from an input data set at a time, and the maximum perturbation that a single data record in the input data set can cause to a query output is determined, thereby determining the sensitivity.

In the brute-force analysis algorithm, each time one data record is deleted from the input data set, and the maximum perturbation that a data record can cause to the output is determined. As a result, when the input data set has a relatively large quantity of data records, a relatively large calculation workload is incurred in determining the sensitivity, resulting in low efficiency in data processing.

### SUMMARY

This application provides a data processing method and apparatus, a computing device for data processing, and a storage medium, to improve efficiency in data processing.

According to a first aspect, this application provides a data processing method. The method includes: determining, based on a query algorithm, a first query output corresponding to an input data set; determining, based on a second query output of an unsampled data record in the input data set and the query algorithm, a perturbation of each of a target quantity of sampled data records in the input data set to the first query output, to obtain sensitivity corresponding to the input data set, where the unsampled data record and the target quantity of data records constitute the input data set; and adding noise to the first query output based on the sensitivity and outputting a noised first query output.

In the solution described in this application, the data processing method is performed by a data processing apparatus. After receiving a data query request, the data processing apparatus may determine an input data set corresponding to the data query request. The data processing apparatus may input the input data set to a query algorithm corresponding to the query request, to obtain a first query output corresponding to the input data set. The data processing apparatus inputs an unsampled data record in the input data set to the query algorithm, where an obtained output is a second query output. The data processing apparatus determines, by using the second query output and the query algorithm, a perturbation of each of a target quantity of sampled data records in the input data set to the first query output. The data processing apparatus determines the maximum perturbation among all perturbations as sensitivity corresponding to the input data set. The data processing apparatus adds noise (where the noise may be Laplace noise) to the first query output based on the sensitivity, to obtain a noised first query output. The data processing apparatus sends the first query output to a terminal used by a data inquirer. In this way, the sensitivity of the input data set can be accurately determined by using only the target quantity of data records, and there is no need to determine a perturbation of each data record in the input data set to the query output. Therefore, the sensitivity can be rapidly determined, and the query output can be rapidly output to the data inquirer, thereby improving efficiency in data processing. In addition, the target quantity is fixed, so that a computation workload for determining sensitivity remains the same regardless of a size of a data set. This is unlike the case with a brute-force analysis algorithm, in which a computation workload rapidly increases as a size of an input data set increases. Therefore, as a quantity of data records in a data set increases, overheads in calculating sensitivity in this application are smaller relative to overheads in calculating sensitivity by using the brute-force analysis algorithm.

In a possible implementation, before the determining, based on a query algorithm, a first query output corresponding to an input data set, the method further includes: receiving a data query request, where the data query request includes the query algorithm; and randomly sampling a target quantity of data records from the input data set corresponding to the data query request, to obtain the target quantity of data records and the unsampled data record in the input data set.

In the solution described in this application, when the data inquirer wants to query data in the data processing apparatus, the data inquirer inputs query content and the query algorithm to be used in the terminal used. The terminal may generate the data query request and include the query algorithm and the query content in the data query request. The query content is used to determine the input data set. The terminal sends the data query request to the data processing apparatus. The data processing apparatus receives the data query request and obtains the query algorithm and the query content from the data query request through parsing. Then the data processing apparatus determines, by using the query content, the input data set corresponding to the data query request. The data processing apparatus randomly samples a target quantity of data records from the input data set. The target quantity of data records are sampled data records, and a data record other than the target quantity of data records in the input data set may be referred to as an unsampled data record. In this way, the data processing apparatus can determine the sampled data records and the unsampled data record.

In a possible implementation, the determining, based on a query algorithm, a first query output corresponding to an input data set includes: determining, based on the query algorithm, the second query output corresponding to the unsampled data record and a third query output corresponding to the target quantity of data records; and determining, based on the second query output and the third query output, the first query output corresponding to the input data set.

In the solution described in this application, the data processing apparatus may input the unsampled data record to the query algorithm, where an obtained output is the second query output. The data processing apparatus may input the sampled data records (namely the target quantity of data records) to the query algorithm, where an obtained output is a third query output. The data processing apparatus obtains the first query output by inputting the second query output and the third query output to the query algorithm. In this way, because of associativity and commutativity of a big data operator in the query algorithm, the first query output can be rapidly determined by using the second query output and the third query output.

In a possible implementation, the determining, based on a second query output of an unsampled data record in the input data set and the query algorithm, a perturbation of each of a target quantity of sampled data records in the input data set to the first query output, to obtain sensitivity corresponding to the input data set includes: determining, based on the query algorithm, the third query output corresponding to the target quantity of data records; determining, based on the query algorithm, a query output provided after each of the target quantity of data records is deleted; determining the perturbation of each of the target quantity of sampled data records in the input data set to the first query output based on the second query output of the unsampled data record, the third query output, and the query output provided after each of the target quantity of data records is deleted; and determining, as the sensitivity corresponding to the input data set, the maximum perturbation among the perturbations of the target quantity of data records to the first query output.

In the solution described in this application, the data processing apparatus may input the sampled data records (namely the target quantity of data records) to the query algorithm, where the obtained output is the third query output. Then the data processing apparatus deletes the target quantity of data records one at a time, and determines a query output provided after each data record is deleted. The data processing apparatus inputs, to the query algorithm, the second query output and the query output provided after each of the target quantity of data records is deleted, to obtain query outputs respectively corresponding to the query outputs provided after the target quantity of data records are deleted one at a time. The query outputs respectively corresponding to the query outputs provided after the target quantity of data records are deleted one at a time are subsequently referred to as a plurality of query outputs corresponding to the target quantity of data records. The data processing apparatus separately subtracts each of the plurality of query outputs corresponding to the target quantity of data records from the first query output, to obtain the perturbation of each of the target quantity of data records to the first query output.

Then the data processing apparatus determines the maximum perturbation among the perturbations of the target quantity of data records to the first query output, and determines the maximum perturbation as the sensitivity corresponding to the input data set. In this way, the sensitivity of the input data set can be accurately determined by using only the target quantity of data records, and there is no need to determine a perturbation of each data record in the input data set to the first query output. Therefore, the sensitivity can be rapidly determined, and the query output can be rapidly output to the data inquirer, thereby improving efficiency in data processing.

In a possible implementation, before the adding noise to the first query output based on the sensitivity and outputting a noised first query output, the method further includes: splitting the input data set into at least two partitions based on partitions to which the data records belong; determining current query outputs of the at least two partitions based on the query algorithm; and determining a difference between the current query output and a historical query output of each of the at least two partitions.

In the solution described in this application, before the noised first query output is output, whether the input data set constitutes a differential attack is further determined. If the input data set does not constitute a differential attack, the noised first query output is directly output. This can prevent differential attacks as far as possible.

In a possible implementation, the method further includes: for a target partition of the at least two partitions, if a current query output of the target partition is the same as a historical query output of the target partition, deleting at least one data record from the target partition, so that a current query output and the historical query output of each of the at least two partitions are different; determining, based on the query algorithm, a query output provided after the at least one data record is deleted from the target partition; determining, based on the query output provided after the at least one data record is deleted from the target partition, a fourth query output of the input data set; and adding noise to the fourth query output based on the sensitivity and outputting a noised fourth query output.

In the solution provided in this application, before the noised first query output is output, whether the input data set constitutes a differential attack is further determined. If the input data set constitutes a differential attack, data records in a partition are adjusted so that the input data set does not constitute a differential attack, and a query output is recalculated. In this way, when the input data set constitutes a differential attack, the differential attack can be prevented. Therefore, protection of data privacy can be implemented as far as possible.

According to a second aspect, this application provides a data processing apparatus. The apparatus includes one or more modules, which are configured to implement the method according to any one of the first aspect or the possible implementations of the first aspect.

According to a third aspect, a computing device for data processing is provided. The computing device includes a processor and a memory. The memory stores computer instructions, and the processor executes the computer instructions to implement the method according to the first aspect or the possible implementations of the first aspect.

According to a fourth aspect, a computer readable storage medium is provided. The computer readable storage medium stores computer instructions. When the computer instructions in the computer readable storage medium are executed by a computing device, the computing device is enabled to perform the method according to the first aspect or the possible implementations of the first aspect, or the computing device is enabled to implement a function of the apparatus according to the second aspect or the possible implementations of the second aspect.

According to a fifth aspect, a computer program product including instructions is provided. When the computer program product runs on a computing device, the computing device is enabled to perform the method according to the first aspect or the possible implementations of the first aspect, or the computing device is enabled to implement a function of the apparatus according to the second aspect or the possible implementations of the second aspect.

### BRIEF DESCRIPTION OF DRAWINGS

FIG. 1 is a schematic diagram of associativity of a big data operator according to an example embodiment of this application;
FIG. 2 is a schematic diagram of commutativity of a big data operator according to an example embodiment of this application;
FIG. 3 is a schematic diagram of a structure of a computing device according to an example embodiment of this application;
FIG. 4 is a schematic diagram of a system of a data processing method according to an example embodiment of this application;
FIG. 5 is a schematic diagram of a structure of a data processing apparatus according to an example embodiment of this application;
FIG. 6 is a schematic flowchart of a data processing method according to an example embodiment of this application;
FIG. 7 is a schematic diagram of determining a query output according to an example embodiment of this application;
FIG. 8 is a schematic diagram of a differential attack according to an example embodiment of this application;
FIG. 9 is a schematic diagram of independently processing a partition according to an example embodiment of this application;
FIG. 10 is a schematic flowchart of a data processing method according to an example embodiment of this application;
FIG. 11 is a schematic diagram of determining a query output according to an example embodiment of this application;
FIG. 12 is a schematic diagram of determining a query output according to an example embodiment of this application;
FIG. 13 is a schematic diagram of a structure of a data processing apparatus according to an example embodiment of this application;
FIG. 14 is a schematic diagram of a structure of a data processing apparatus according to an example embodiment of this application; and
FIG. 15 is a schematic diagram of a structure of a data processing apparatus according to an example embodiment of this application.

### DESCRIPTION OF EMBODIMENTS

To make objectives, technical solutions, and advantages of this application clearer, the following further describes implementations of this application in detail with reference to the accompanying drawings.

To facilitate understanding embodiments of this application, the following first describes concepts of terms involved in embodiments of this application.

A big data processing system is a program, for example, Apache Spark, Camdoop, DryadLINQ, or Pregel, that computes a massive amount of data in parallel by using a plurality of devices.

A data record is a row of data information in a data set. Data records are units that constitute a data set.

A big data operator is a function that has associativity and commutativity, for example, a map operator (map) and a reduce operator (reduce) in Apache Spark.

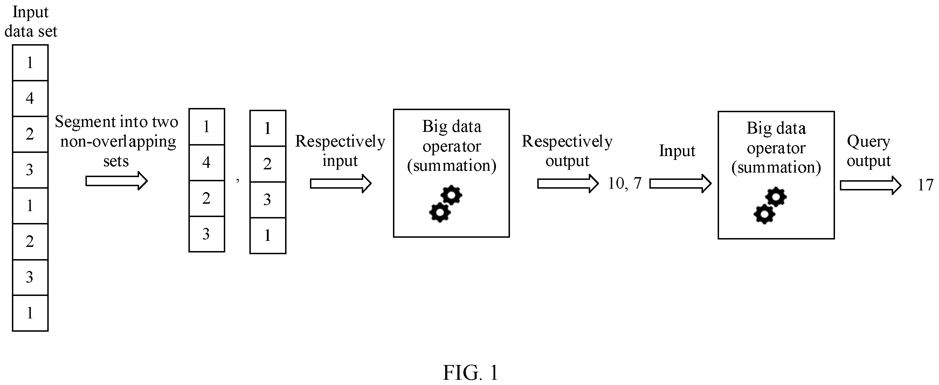

Associativity: When data records in an input data set are segmented into a plurality of non-overlapping sets, a big data operator may first compute an output of each set, and then the outputs of all the sets are used as an input of the big data operator to obtain an output, which equals an output obtained by inputting the input data set to the big data operator. For example, as shown in FIG. 1, a big data operator is used for summation. An output obtained by inputting an input data set ({1,4,2,3,1,2,3,1}) to the big data operator is 17. The input data set ({1,4,2,3,1,2,3,1}) is segmented into two non-overlapping sets (which are {1,4,2,3} and {1,2,3,1}). An output obtained by inputting {1,4,2,3} to the big data operator is 10, and an output obtained by inputting {1,2,3,1} to the big data operator is 7. An output obtained by inputting 10 and 7 to the big data operator is 17.

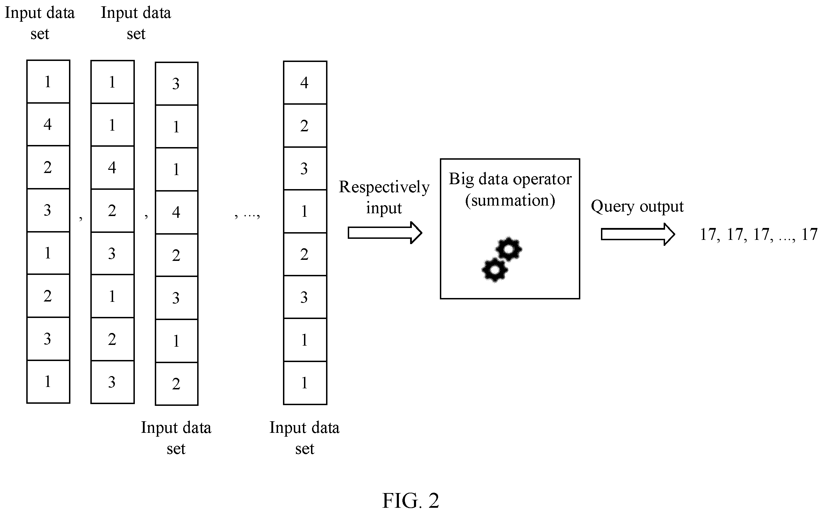

Commutativity: A big data operator allows records of an input data set to be input to the big data operator in any order. Outputs of the big data operator remain the same regardless of an order in which the data records of the input data set are input to the big data operator. For example, as shown in FIG. 2, a big data operator is used for summation, and an input data set is {1,4,2,3,1,2,3,1}. The input data set can be input to the big data operator in any order, for example, {1,4,2,3,1,2,3,1}, {1,1,4,2,3,1,2,3}, {3,1,1,4,2,3,1,2}, ..., or {4,2,3,1,2,3,1,1}, and an output is always 17.

A map operator is one of commonly used operators in Apache Spark and is a one-to-one function that has associativity and commutativity. A one-to-one function is a function for which both an input and an output have only one data record. A major application of the map operator is to change a value or a value type of a single data record, for example, to convert a character string into a number.

A reduce operator is one of commonly used operators in Apache Spark and is a many-to-one function that has associativity and commutativity. A many-to-one function is a function for which an input has a plurality of data records and an output has only one value. A major application of the reduce operator is to combine values of a plurality of data records, for example, to calculate a sum of values of a plurality of data sets to obtain one output.

Query algorithm: A query algorithm includes a big data operator, for example, the map operator and the reduce operator. The query algorithm may be any big data algorithm, for example, an algorithm for summation or an algorithm for subtraction.

Sensitivity is the maximum change to a query output after a data record is added to or deleted from an input data set (where the query output is an output obtained by inputting the input data set to a query algorithm).

In the big data field, to implement privacy protection for personal data, noise is added to a query output based on sensitivity. The noise is determined based on sensitivity of an input data set. Therefore, how to quickly and accurately determine sensitivity is of crucial importance. In a related technology, a brute-force analysis algorithm is used to determine the sensitivity. Specific processing is as follows: One data record is deleted from an input data set at a time, and the maximum perturbation that a single data record in the input data set can cause to a query output is determined, thereby determining the sensitivity. In the brute-force analysis algorithm, each time one data record is deleted from the input data set, and the maximum perturbation that a data record can cause to the output is determined. As a result, when the input data set has a relatively large quantity of data records, a relatively large calculation workload is incurred in determining the sensitivity, resulting in low efficiency in data processing. Therefore, this application provides a data processing method, to rapidly and accurately determine sensitivity, thereby implementing efficient data processing.

Before the data processing method provided in embodiments of this application is described, a system architecture to which embodiments of this application are applicable is described first.

The data processing method may be performed by a data processing apparatus. The data processing apparatus may be a hardware apparatus, such as a server or a terminal computing device, or may be a software apparatus (for example, may be a software program that runs on a hardware apparatus).

The data processing apparatus may be deployed in a cloud environment. The cloud environment is an entity that provides users with cloud services by using infrastructure resources in cloud computing mode. The cloud environment includes a cloud data center and a cloud service platform. The cloud data center includes massive infrastructure resources (including computing resources, storage resources, and network resources) owned by a cloud service provider. Computing resources included by the cloud data center may be a large quantity of computing devices (for example, servers). The data processing apparatus may be a server for data processing in the cloud data center. The data processing apparatus may alternatively be a virtual machine for data processing that is created in the cloud data center. The data processing apparatus may alternatively be a server deployed in the cloud data center or a software apparatus deployed on a virtual machine. The software apparatus is used for data processing. The software apparatus may be deployed on a plurality of servers in a distributed manner, deployed on a plurality of virtual machines in a distributed manner, or deployed on a virtual machine and a server in a distributed manner.

When the data processing apparatus is a software apparatus, the data processing apparatus may be logically divided into a plurality of parts, with each part providing a different function. (For example, the data processing apparatus may include a determining module and an output module.) The plurality of parts of the data processing apparatus may be separately deployed in different environments or devices. The parts of the data processing apparatus that are deployed in different environments or devices collaborate with each other to implement data processing. It should be understood that this application does not impose a restrictive limitation on division of the parts of the data processing apparatus, or impose a restrictive limitation on an environment in which the data processing apparatus is specifically deployed. In actual application, the data processing apparatus may be adaptively deployed based on a computing capability of each computing device or a specific application requirement.

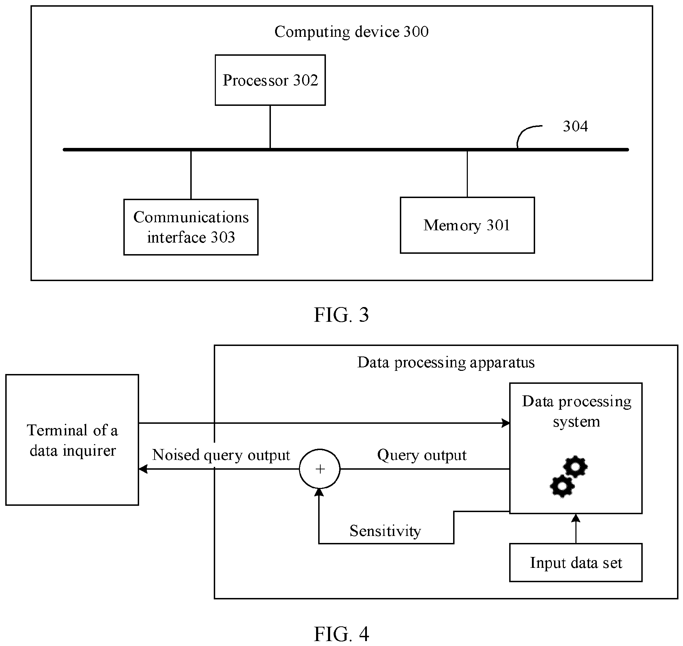

When the data processing apparatus is a software apparatus, the data processing apparatus may alternatively be independently deployed on a computing device in any environment (such as a cloud environment or a terminal computing device). When the data processing apparatus is a hardware device, the data processing apparatus may be a computing device 300 in any environment. FIG. 3 is a schematic diagram of a structure of a computing device 300. The computing device 300 shown in FIG. 3 includes a memory 301, a processor 302, a communications interface 303, and a bus 304. The memory 301, the processor 302, and the communications interface 303 are communicatively connected to each other by using the bus 304.

The memory 301 may be a read-only memory (Read-Only Memory, ROM), a static storage device, a dynamic storage device, or a random access memory (Random Access Memory, RAM). The memory 301 may store computer instructions. When the computer instructions stored in the memory 301 are executed by the processor 302, the processor 302 and the communications interface 303 are configured to perform a data processing method. The memory may further store data. For example, a part of the memory 301 is configured to store data required by the data processing method and configured to store intermediate data during execution of a program or result data.

The processor 302 may use a general-purpose central processing unit (Central Processing Unit, CPU), an application-specific integrated circuit (Application-Specific Integrated Circuit, ASIC), a graphics processing unit (graphics processing unit, GPU), or any combination thereof. The processor 302 may include one or more chips. The processor 302 may include an artificial intelligence (artificial intelligence, AI) accelerator, for example, a neural processing unit (neural processing unit, NPU).

The communications interface 303 uses, by way of example rather than limitation, a transceiver module such as a transceiver to implement communication between the computing device 300 and another device or a communication network. For example, the communications interface 303 may be used to obtain data required in data processing.

The bus 304 may include a path for transferring information between various parts (such as the memory 301, the processor 302, and the communications interface 303) of the computing device 300.

To facilitate better understanding of embodiments of this application, as shown in FIG. 4, an entire data query process is provided. To be specific, a terminal of a data inquirer sends a query request to a data processing apparatus, where the query request includes a query algorithm. The data processing apparatus may calculate a query output and sensitivity of an input data set, add noise to the query output based on the sensitivity, and return a noised query output to the terminal of the data inquirer.

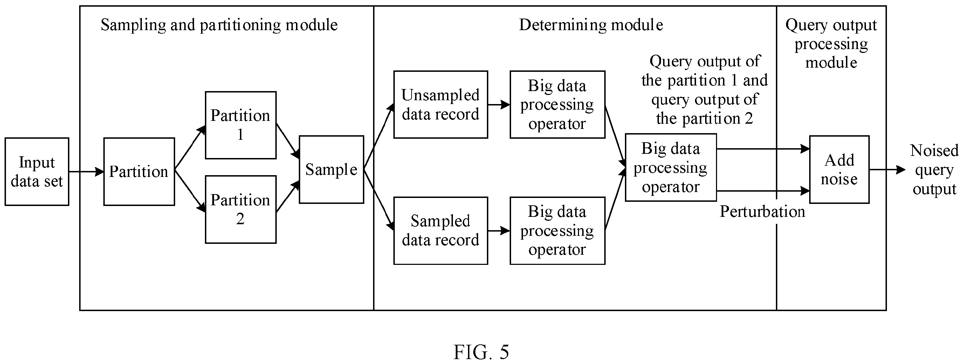

When performing a data processing method, a data processing apparatus is logically divided into three modules, which are respectively a sampling and partitioning module, a module for determining sensitivity and a query output of a partition (which may be referred to as a determining module for short), and a query output processing module (which may be referred to as an output module for short). The determining module may include a big data operator. For example, as shown in FIG. 5, the determining module may include three big data operators.

The sampling and partitioning module is configured to split an input data set into partitions and randomly sample a target quantity of data records from the input data set, so that the input data set is divided into sampled data records and an unsampled data record.

The determining module is configured to determine a perturbation of each of the sampled data record to a query output of the input data set and determine query outputs of the partitions.

The output module is configured to add noise to the query output of the input data set, and the like.

Certainly, the data processing apparatus may further include a receiving module, configured to receive a data query request and the like.

It should be noted that a query algorithm includes a big data operator and the determining module also includes big data operators. When the data processing method is performed, actually the big data operators in the determining module jointly implement the query algorithm.

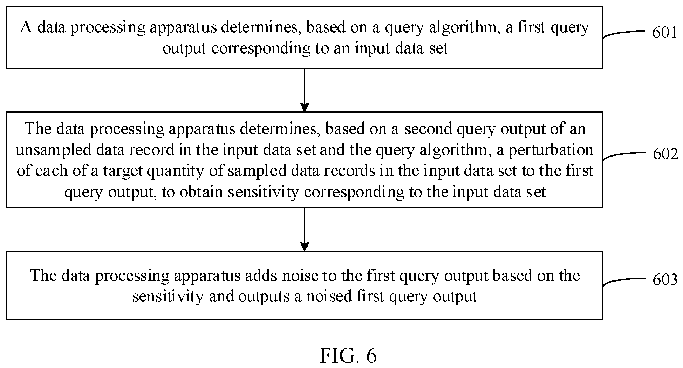

The following describes a data processing method provided in an embodiment of this application with reference to FIG. 6. The method may be performed by a data processing apparatus. As shown in FIG. 6, a processing process of the method is as follows:

Step 601. The data processing apparatus determines, based on a query algorithm, a first query output corresponding to an input data set.

In this embodiment, after receiving a data query request, the data processing apparatus may determine an input data set corresponding to the data query request. The data processing apparatus may input the input data set to a query algorithm corresponding to the query request, to obtain a first query output corresponding to the input data set.

For example, the query algorithm corresponding to the query request is an algorithm for summation, the input data set is {1,4,2,3,1,2,3,1}, and the query output is 17.

Step S602. The data processing apparatus determines, based on a second query output of an unsampled data record in the input data set and the query algorithm, a perturbation of each of a target quantity of sampled data records in the input data set to the first query output, to obtain sensitivity corresponding to the input data set.

The target quantity is a relatively large number, for example, 1000. The target quantity of data records include sampled (also referred to as selected) data records in the input data set. The unsampled data record is a data record other than the target quantity of data records in the input data set.

In this embodiment, the data processing apparatus may input the unsampled data record in the input data set to the query algorithm, where an obtained output is the second query output. The data processing apparatus determines, by using the second query output and the query algorithm, the perturbation of each of the target quantity of sampled data records in the input data set to the first query output. The data processing apparatus determines the maximum perturbation among all perturbations as the sensitivity corresponding to the input data set.

Step 603. The data processing apparatus adds noise to the first query output based on the sensitivity and outputs a noised first query output.

In this embodiment, the data processing apparatus may add noise (where the noise may be Laplace noise) to the first query output based on the sensitivity, to obtain the noised first query output. The data processing apparatus sends the first query output to a terminal used by a data inquirer. Specifically, when the sensitivity is low, a small amount of noise is added to the first query output; and when the sensitivity is high, a large amount of noise is added to the first query output.

In this way, the sensitivity of the input data set can be accurately determined by using only the target quantity of data records, and there is no need to determine a perturbation of each data record in the input data set to the query output. Therefore, the sensitivity can be rapidly determined, and the query output can be rapidly output to the data inquirer. In addition, the target quantity is fixed, so that a computation workload for determining sensitivity remains the same regardless of a size of a data set. This is unlike the case with a brute-force analysis algorithm, in which a computation workload rapidly increases as a size of an input data set increases. Therefore, as a quantity of data records in a data set increases, overheads in calculating sensitivity in this application are smaller relative to overheads in calculating sensitivity by using the brute-force analysis algorithm.

The sensitivity of the input data set can be accurately determined for the following reason:

A perturbation of a single data record to a query output approximately conforms to normal distribution (where perturbations of most data records to the query output are small, and perturbations of a small part of data records to the query output are large). For an input data set that conforms to normal distribution, a target quantity (for example, 1000) of samples randomly sampled from the input data set are sufficient to accurately fit the normal distribution to which the input data set conforms, regardless of a size of the input data set. Therefore, for an input data set, sensitivity estimated in this application is the same as actual sensitivity in theory of statistics.

In a possible implementation, before step 601, the following processing is further performed:

Step 600. The data processing apparatus receives the data query request, where the data query request includes the query algorithm; and randomly samples the target quantity of data records from the input data set corresponding to the data query request, to obtain the target quantity of data records and the unsampled data record in the input data set.

In this embodiment, when the data inquirer wants to query data in the data processing apparatus, the data inquirer inputs query content and the query algorithm to be used in the terminal used. The terminal may generate the data query request and include the query algorithm and the query content in the data query request, where the query content is used to determine the input data set, for example, information about patients hospitalized from May 10, 2020 to May 30, 2020. The query algorithm here may be specific algorithm execution code or may be an identifier of the query algorithm.

The terminal sends the data query request to the data processing apparatus. The data processing apparatus receives the data query request and obtains the query algorithm and the query content from the data query request through parsing. Then the data processing apparatus determines, by using the query content, the input data set corresponding to the data query request. The data processing apparatus randomly samples the target quantity of data records from the input data set. The target quantity of data records are sampled data records, and a data record other than the target quantity of data records in the input data set may be referred to as an unsampled data record. In this way, the data processing apparatus can determine the sampled data records and the unsampled data record. In addition, if the query algorithm in the query request is execution code, the data processing apparatus directly runs the execution code subsequently. If the query algorithm in the query request is an identifier of the query algorithm, the data processing apparatus obtains execution code of the query algorithm by using the identifier and directly runs the execution code subsequently.

In addition, in step 600, when the terminal of the data inquirer sends the query request to the data processing apparatus, the query request further includes an identifier of the data inquirer. When receiving the data query request, the data processing apparatus may obtain the identifier of the data inquirer by parsing the data query request. The data processing apparatus determines whether the identifier is included in a trusted identifier list (where when a data inquirer in the trusted identifier list initiates a query, a result is directly output, without the need to add noise). If the identifier of the data inquirer is included in the trusted identifier list, a query output of the input data set is determined and directly output. If the identifier of the data inquirer is not included in the trusted identifier list, a processing process of adding noise is performed.

In a possible implementation, the first query output may be determined by using associativity and commutativity of the big data operator. Processing in step 601 is as follows:

The data processing apparatus determines, based on the query algorithm, the second query output corresponding to the unsampled data record and a third query output corresponding to the target quantity of data records; and determines, based on the second query output and the third query output, the first query output corresponding to the input data set.

In this embodiment, the data processing apparatus may input the unsampled data record to the query algorithm, where an obtained output is the second query output. The data processing apparatus may input the sampled data records (namely the target quantity of data records) to the query algorithm, where an obtained output is the third query output. The data processing apparatus obtains the first query output by inputting the second query output and the third query output to the query algorithm.

For example, an input data set is {1,4,2,3,1,2,3,1}, a target quantity of data records are {1,4,2}, unsampled data records are {3,1,2,3,1}, and a query algorithm is used for summation. The data processing apparatus inputs {1,4,2} to the query algorithm, and an output is 7 (namely the third query output). The data processing apparatus inputs {3,1,2,3,1} to the query algorithm, and an output is 10 (namely the second query output). The data processing apparatus inputs 10 and 7 to the query algorithm, and an output is 17 (namely the first query output).

In a possible implementation, in step 602, processing of determining the sensitivity corresponding to the input data set is as follows:

The data processing apparatus determines, based on the query algorithm, the third query output corresponding to the target quantity of data records; determines, based on the query algorithm, a query output provided after each of the target quantity of data records is deleted; determines the perturbation of each of the target quantity of sampled data records to the first query output based on the second query output of the unsampled data record, the third query output, and the query output provided after each of the target quantity of data records is deleted; and determines, as the sensitivity corresponding to the input data set, the maximum perturbation among the perturbations of the target quantity of data records to the first query output.

In this embodiment, the data processing apparatus may input the sampled data records (namely the target quantity of data records) to the query algorithm, where an obtained output is the third query output. Then the data processing apparatus deletes the target quantity of data records one at a time, and determines a query output provided after each data record is deleted.

The data processing apparatus obtains the first query output by inputting the second query output of the unsampled data record and the third query output to the query algorithm. The data processing apparatus inputs, to the query algorithm, the second query output and the query output provided after each of the target quantity of data records is deleted, to obtain query outputs respectively corresponding to the query outputs provided after the target quantity of data records are deleted one at a time. The query outputs respectively corresponding to the query outputs provided after the target quantity of data records are deleted one at a time are subsequently referred to as a plurality of query outputs corresponding to the target quantity of data records. The data processing apparatus separately subtracts each of the plurality of query outputs corresponding to the target quantity of data records from the first query output, to obtain the perturbation of each of the target quantity of data records to the first query output.

Then the data processing apparatus determines the maximum perturbation among the perturbations of the target quantity of data records to the first query output, and determines the maximum perturbation as the sensitivity corresponding to the input data set. In this way, the sensitivity of the input data set can be accurately determined by using only the target quantity of data records, and there is no need to determine a perturbation of each data record in the input data set to the query output. Therefore, the sensitivity can be rapidly determined, and the query output can be rapidly output to the data inquirer.

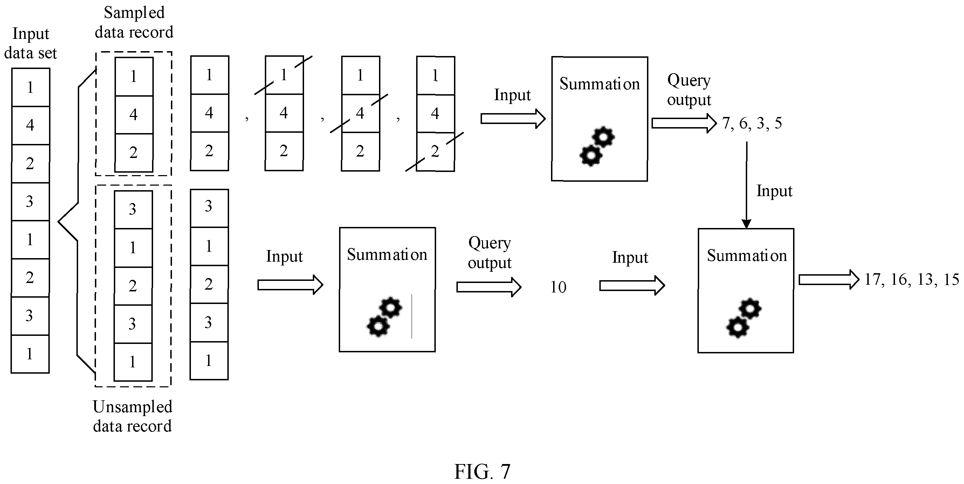

For example, as shown in FIG. 7, an input data set is {1,4,2,3,1,2,3,1}, a target quantity of data records are {1,4,2}, unsampled data records are {3,1,2,3,1}, and a query algorithm is used for summation. The data processing apparatus inputs {1,4,2} to the query algorithm, and an output is 7 (namely the third query output). The data processing apparatus separately inputs {4,2}, { 1,2}, and { 1,4} to the query algorithm, and outputs are respectively 7, 6, 3, and 5. The data processing apparatus inputs {3,1,2,3,1} to the query algorithm, and an output is 10 (namely the second query output). The data processing apparatus inputs 10 and 7 to the query algorithm, and an output is 17 (namely the first query output). A perturbation of the data record 1 to the first query output is 17-16=1, a perturbation of the data record 2 to the first query output is 17-15=2, and a perturbation of the data record 4 to the first query output is 17-13=4. Therefore, the maximum perturbation is 4, and sensitivity of the input data set {1,4,2,3,1,2,3,1} is 4. In FIG. 7, a slash on a data record indicates to delete the data record.

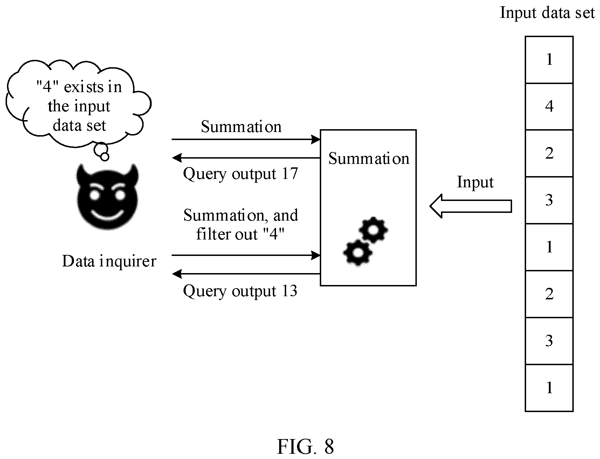

In a possible implementation, even if the sensitivity estimated in the process shown in FIG. 6 deviates from actual sensitivity, in this embodiment of this application, differential privacy protection can be provided for the input data set as far as possible by detecting and preventing a differential attack. The following first describes differential attack. Given two query algorithms Q and Q' (where input data sets corresponding to Q and Q' are respectively D and D'), "differential attack" is defined as follows: The query algorithms Q and Q' have a same input-output mapping, and D and D' differ from each other by only one data record. As shown in FIG. 8, a data inquirer submits two algorithms with the same input-output mapping (which is summation here) to a data processing apparatus, and demands that a data record (4) in an input data set be filtered out when submitting a query algorithm Q'. An input data set D {1,4,2,3,1,2,3,1} of a query algorithm Q and an input data set D' {1,2,3,1,2,3,1} of a query algorithm Q' differ from each other by only one data record (4). The data inquirer may determine, based on a difference between query outputs of the two query algorithms (where a difference between query outputs 17 and 13 is 4), that the data record "4" exists in the input data set.

To better prevent a differential attack, a principle for detecting and preventing a differential attack in embodiments of this application also uses associativity and commutativity of a big data operator. The associativity and commutativity of the big data operator enable each data record in an input data set or each subset (namely partitions to be mentioned later) of an input data set to be processed independently. Therefore, when a query output of a data record or partition changes, a query output of another record or partition is not affected. Specifically, given an input data set D, D1 (a partition 1) and D2 (a partition 2) are two non-overlapping partitions of D, and a union of D1 and D2 is D (namely D1∪D2=D). Aquery output of D1 is O1=Q(D1), and a query output of D2 is O2=Q(D2). If one data record is missing from D1, O1 may change, but O2 definitely does not change.

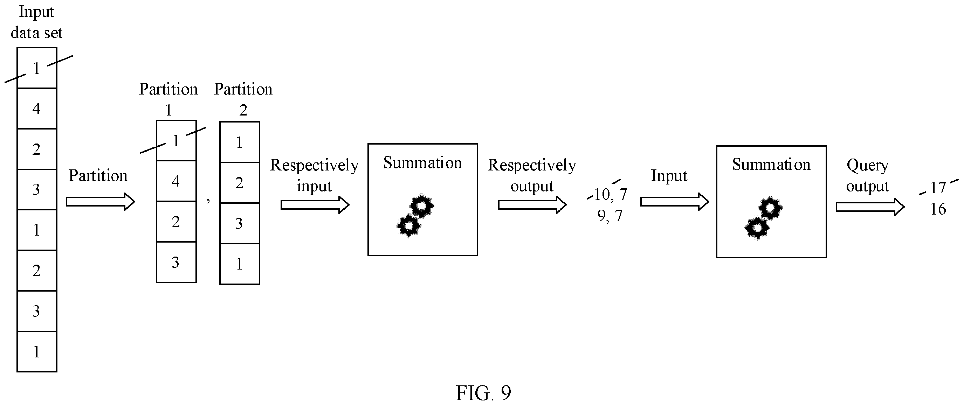

For example, as shown in FIG. 9 (where a slash in FIG. 9 means deletion), it is assumed that an input data set is {1,4,2,3,1,2,3,1}, that the first four data records of the input data set constitute a partition 1 (in other words, D1 is {1,4,2,3}), that the last four data records of the input data set constitute a partition 2 (in other words, D2 is {1,2,3,1}), and that a query algorithm is an algorithm for summation. If the first data record is deleted from D1, a query output of D1 decreases from 10 to 9, but a query output of D2 remains unchanged (where the query output is still 7), and a query output of the input data set decreases from 17 to 16. This is because associativity and commutativity of a big data operator enable each partition to be processed independently. Therefore, in embodiments of this application, a query output of a current query on a partition (namely a current query output) and a query output of a previous query on the partition (namely a historical query output) are compared, to determine whether the current query output can constitute a differential attack, (specifically, to determine whether query algorithms of the current query and the previous query have the same input-output mapping and whether input data sets differ by only one record).

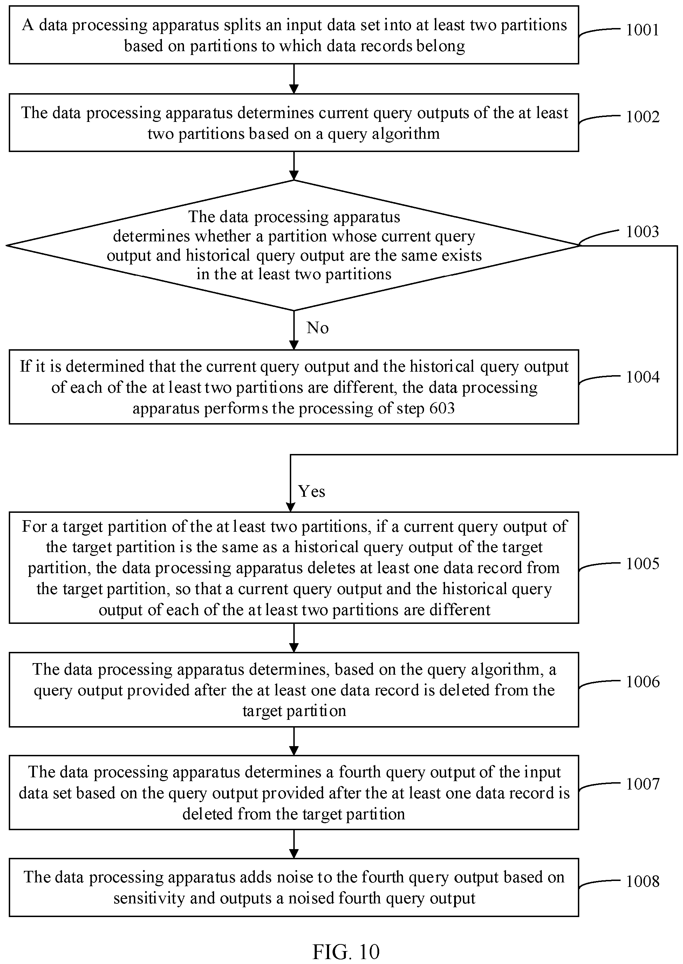

The following describes a process of preventing a differential attack during data processing, as shown in FIG. 10.

Step 1001. A data processing apparatus splits an input data set into at least two partitions based on partitions to which data records belong.

In this embodiment, partitions have been obtained through splitting when the data processing apparatus stores a data record. Alternatively, partitions have been obtained through splitting when a device storing the input data set stores a data record. A partition is assigned to each data record when the data record is stored. In other words, an identifier of a partition to which each data record belongs is stored for the data record. Specifically, all partitions usually include basically the same quantity of data records during partition splitting.

The data processing apparatus may split the input data set into at least two partitions based on a partition to which each data record in the input data set belongs.

In a possible implementation, to make processing of preventing a differential attack occupy a relatively small amount of processing resources, two partitions are obtained through splitting the input data set.

Step 1002. The data processing apparatus determines current query outputs of the at least two partitions based on a query algorithm.

In this embodiment, the data processing apparatus separately inputs data records of the at least two partitions to the query algorithm, where obtained outputs are the current query outputs of the at least two partitions.

Step 1003. The data processing apparatus determines whether a partition whose current query output and historical query output are the same exists in the at least two partitions.

In this embodiment, each time when the data processing apparatus determines a query output of a partition, the data processing apparatus stores the query output, which may be stored locally, may be stored in another externally connected device, or the like. The data processing apparatus obtains a historical query output of each of the at least two partitions. For each partition, the data processing apparatus determines whether the current query output and the historical query output of each partition are the same. For example, at least two partitions included in an input data set D are D1 and D2, whose current query outputs are respectively O1 and O2 and whose historical query outputs are respectively O1' and O2'. Whether O1 and O1' are the same is determined through comparison, and whether O2 and O2' are the same is determined through comparison. If O1 and O1' are different and O2 and O2' are different, D1 and D2 need to differ by two or more data records, and it is impossible to constitute a differential attack. In this case, go to step 1004. If O1 and O1' are the same and/or O2 and O2' are the same, a current query output and a historical query output of the input data set may constitute a differential attack. In this case, go to step 1005.

It should be noted that because the partitions have been obtained through splitting when a data record is stored, only a data record in a partition is updated in a subsequent process of updating the input data set, without causing a change to a quantity of partitions of the input data set. Therefore, it is possible that a current query output and a historical query output of a partition are different.

Step 1004. If it is determined that the current query output and the historical query output of each of the at least two partitions are different, the data processing apparatus performs the processing of step 603.

In this embodiment, when the data processing apparatus determines that the current query output and the historical query output of each of the at least two partitions are different, the data processing apparatus may determine that the input data set does not constitute a differential attack and performs the processing of step 603. To be specific, the data processing apparatus adds noise to a first query output based on sensitivity, obtains a noised first query output, and sends the first query output to a terminal used by a data inquirer.

In addition, if the partitions do not have historical query outputs, the input data set does not constitute a differential attack, and the processing of step 603 may be directly performed.

Step 1005. For a target partition of the at least two partitions, if a current query output of the target partition is the same as a historical query output of the target partition, the data processing apparatus deletes at least one data record from the target partition, so that a current query output and the historical query output of each of the at least two partitions are different. Then processing in steps 1006 to step 1008 is performed.

The target partition is a partition, of the at least two partitions, whose current query output and historical query output are the same.

In this embodiment, when determining that the current query output of the target partition is the same as a historical query output of the target partition, the data processing apparatus may delete at least one data record from the target partition, so that a current query output of the target partition is different from each historical query output of the target partition.

It should be noted that to ensure that a query output provided to the data inquirer is relatively accurate, the at least one data record here is one data record.

Step 1006. The data processing apparatus determines, based on the query algorithm, a query output provided after the at least one data record is deleted from the target partition.

In this embodiment, the data processing apparatus inputs, to the query algorithm, the target partition from which the at least one data record is deleted, to obtain the query output. In addition, the data processing apparatus inputs, to the query algorithm, data records of a partition other than the target partition of the at least two partitions, to obtain a query output.

Step 1007. The data processing apparatus determines a fourth query output of the input data set based on the query output provided after the at least one data record is deleted from the target partition.

In this embodiment, an output obtained by the data processing apparatus by inputting the query output obtained in step 1006 to the query algorithm is the fourth query output of the input data set after a differential attack is prevented.

Step 1008. The data processing apparatus adds noise to the fourth query output based on sensitivity and outputs a noised fourth query output.

In this embodiment, the data processing apparatus may add noise to the fourth query output based on the sensitivity obtained in step 602, to obtain the noised fourth query output. The data processing apparatus sends the noised fourth query output to the terminal of the data inquirer.

In this way, whether a differential attack is constituted can be accurately determined. When a differential attack is constituted, the differential attack is prevented, to protect personal data.

In addition, in the process of FIG. 10, the sensitivity in step 602 can still be used because when the input data set includes a relatively large quantity of data records, sensitivity is slightly affected when the at least one data record is deleted from the target partition. Therefore, not only noise added to the query output is not affected, but also data processing time can be reduced.

In addition, in this embodiment of this application, after step 1005 is performed, sensitivity may alternatively be recalculated based on a current input data set, and the sensitivity used in step 1008 is also the recalculated sensitivity.

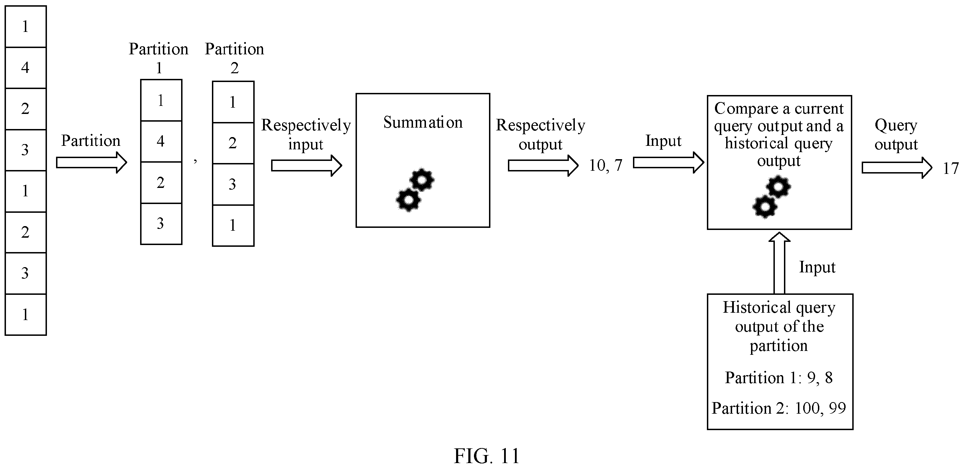

The following example is further provided for the process of FIG. 10. As shown in FIG. 11, a query algorithm is an algorithm for summation, and an input data set is {1,4,2,3,1,2,3,1}. The first four data records of the input data set constitute a partition 1 (in other words, D1 is {1,4,2,3 }), and the last four data records of the input data set constitute a partition 2 (in other words, D2 is {1,2,3,1}). Current query outputs of the partition 1 and the partition 2 are 10 and 7. Historical query outputs of the partition 1 are 9 and 8, and historical query outputs of the partition 2 are 100 and 99. The current query output of the partition 1 is different from the historical query outputs of the partition 1, and the current query output of the partition 2 is different from the historical query outputs of the partition 2. A first query output corresponding to the input data set is 17.

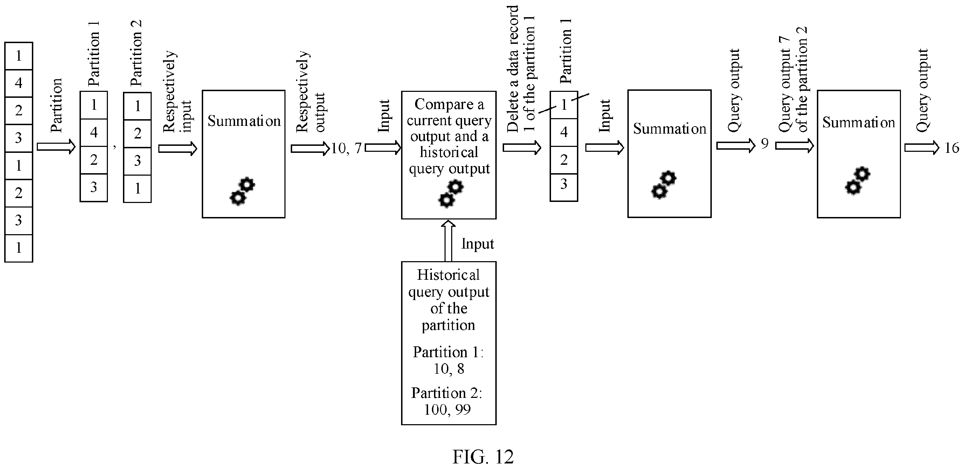

As shown in FIG. 12, a query algorithm is an algorithm for summation, and an input data set is {1,4,2,3,1,2,3,1}. The first four data records of the input data set constitute a partition 1 (in other words, D1 is { 1,4,2,3 }), and the last four data records of the input data set constitute a partition 2 (in other words, D2 is {1,2,3,1}). Current query outputs of the partition 1 and the partition 2 are 10 and 7. Historical query outputs of the partition 1 are 10 and 8, and historical query outputs of the partition 2 are 100 and 99. The current query output of the partition 1 is the same as a historical query output. The data record 1 is deleted from the partition 1, and a current query output changes from 10 to 9. The current query output of the partition 2 is different from the historical query outputs of the partition 2. A fourth query output corresponding to the input data set is 16 (to be specific, 9+7=16).

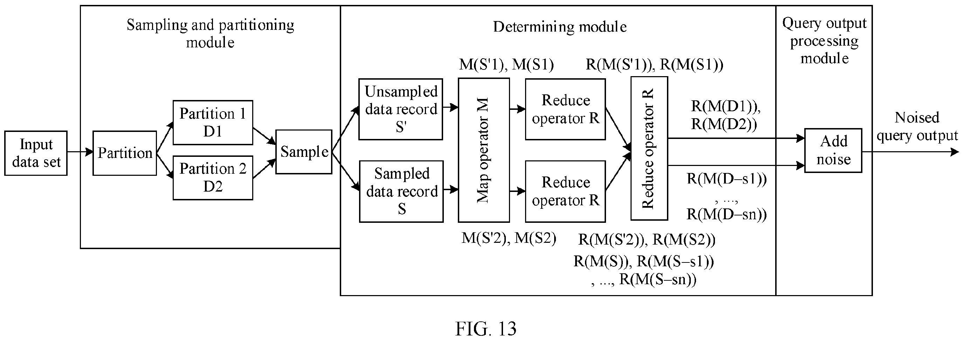

When this application is implemented in Apache Spark, a big data processing system, as shown in FIG. 13, Apache Spark includes a sampling and partitioning module, a module for determining a query output of a partition and sensitivity (referred to as a determining module for short), and a query output processing module (referred to as an output module for short). In the module for determining a query output of a partition and sensitivity, a map operator and a reduce operator may be used for implementation. The sampling and partitioning module implements partitioning and sampling. The determining module calculates a query output of an unsampled data record that belongs to a partition 1, a query output of an unsampled data record that belongs to a partition 2, a query output of a sampled data record that belongs to the partition 1, and a query output of a sampled data record that belongs to the partition 2; and then determines sensitivity and query outputs of the partition 1 and the partition 2 by using the four query outputs. The query output processing module detects and prevents a differential attack by using the sensitivity and the query outputs of the partition 1 and the partition 2, and determines a noised first query output, as mentioned above.

For example, corresponding to FIG. 12, the query algorithm is used for summation. The query algorithm includes a map operator M and a reduce operator R. The input data set is {1,4,2,3,1,2,3,1}. The sampling and partitioning module obtains the input data set and splits the input data set D into the partition 1 (namely D1) and the partition 2 (namely D2). Unsampled data records constitute S' and sampled data records constitute S. S'1 represents unsampled data records that belong to D1, S'2 represents unsampled data records that belong to D2, S1 represents sampled data records that belong to D1, and S2 represents sampled data records that belong to D2.

With reference to FIG. 7 and FIG. 11, S'1 is {3}, S'2 is {1,2,3,1}, S1 is {1,4,2}, and S2 is an empty set. The map operator in the determining module determines that M(S'1)={3}, M(S'2)={1,2,3,1}, M(S1)={1,4,2}, and M(S2)={**Ø**}. The map operator inputs M(S'1)={3} and M(S1)={1,4,2} to a first reduce operator in the determining module, and inputs M(S'2)={1,2,3,1} and M(S2)={**Ø**} to a second reduce operator in the determining module.

A query output R(M(S'1)) of M(S'1) calculated by the first reduce operator in the determining module is 3, a query output R(M(S1)) of M(S1) is 7, and the first reduce operator determines R(M(S)), R(M(S-s1)), ..., and R(M(S-sn)). R(M(S-s1)) is a query output of the sampled data records provided after a data record s1 is deleted from the sampled data records, and R(M(S-sn)) is a query output of the sampled data records provided after a data record sn is deleted from the sampled data records, where n is a quantity of the sampled data records. A query output R(M(S'2)) of M(S'2) calculated by the second reduce operator is 7 and a query output R(M(S2)) of M(S2) is 0. The first reduce operator and the second reduce operator input R(M(S'1)), R(M(S1)), R(M(S'2)), and M(S2) to a third reduce operator in the determining module. The third reduce operator can obtain the following based on associativity and commutativity:
R(M(S'))=R(R(M(S'1)), R(M(S'2)))=10;
R(M(S))=R(R(M(S1)), R(M(S2)))=7;
R(M(D1))=R(R(M(S'1)), R(M(S1)))=10; and
R(M(D2))=R (R(M(S'2)), R(M(S2)))=7.

In addition, the third reduce operator may further determine perturbations of the sampled data records to the input data set based on R(M(S')) and R(M(S)), namely determine R(M(D-s1)), ..., and R(M(D-sn)). R(M(D-s1)) is a perturbation to the input data set after the data record s1 is deleted from the sampled data records, and R(M(D-sn)) is a perturbation to the input data set after the data record sn is deleted from the sampled data records, where n is the quantity of the sampled data records, R(M(D-s1))=R(M(D1))+R(M(D2))-R(M(S-s1)), ..., and R(M(D-sn))=R(M(D1))+R(M(D2))-R(M(S-sn)).

Then the third reduce operator outputs R(M(D1)), R(M(D2)), R(M(D-s1)), ..., and R(M(D-sn)) to the query output processing module.

The query output processing module compares R(M(D1)) with a historical query output of D1, and compares R(M(D2)) with a historical query output of D2. If R(M(D1)) is different from the historical query output of D1, and R(M(D2)) is different from the historical query output of D2, R(M(D1)) and R(M(D2)) are combined, to obtain a query output R(M(D)) of the input data set D. If R(M(D1)) is the same as the historical query output of D1, and R(M(D2)) is different from the historical query output of D2, one data record is deleted from D1, so that a current query output of D1 is different from the historical query output. Then the query output processing module determines a query output R(M(D1)) of D1 provided after the data record is deleted, and obtains a query output R(M(D)) of the input data set D by combining R(M(D1)) provided after the data record is deleted and R(M(D2)). If R(M(D1)) is different from the historical query output of D1, and R(M(D2)) is the same as the historical query output of D2, one data record is deleted from D2, so that a current query output of D2 is different from the historical query output. Then the query output processing module determines a query output R(M(D2)) of D2 provided after the data record is deleted, and obtains a query output R(M(D)) of the input data set D by combining R(M(D1)) and R(M(D2)) that is provided after the data record is deleted. If R(M(D1)) is the same as the historical query output of D1, and R(M(D2)) is the same as the historical query output of D2, one data record is deleted from both D1 and D2, so that a current query output of D 1 provided after the data record is deleted is different from the historical query output of D1, and a current query output of D2 provided after the data record is deleted is different from the historical query output of D2. Then the query output processing module determines query outputs R(M(D 1)) and R(M(D2)) of D 1 and D2 provided after the data records are deleted, and obtains a query output R(M(D)) of the input data set D by combining R(M(D 1)) provided after the data record is deleted and R(M(D2)) provided after the data record is deleted.

The query output processing module determines the maximum value among R(M(D-s1)), ..., and R(M(D-sn)). The maximum value is the sensitivity of the input data set D. Then the query output processing module adds Laplace noise to R(M(D)) to obtain a noised query output, and returns the noised query output to a terminal of a data inquirer.

In addition, in embodiments of this application, to illustrate technical solutions of this application more vividly, an input data set includes a relatively small quantity of data records. In actual processing, an input data set includes a large quantity of data records.

Moreover, in embodiments of this application, a big data operator usually has associativity and commutativity. Therefore, embodiments of this application may be applied to general big data query algorithms.

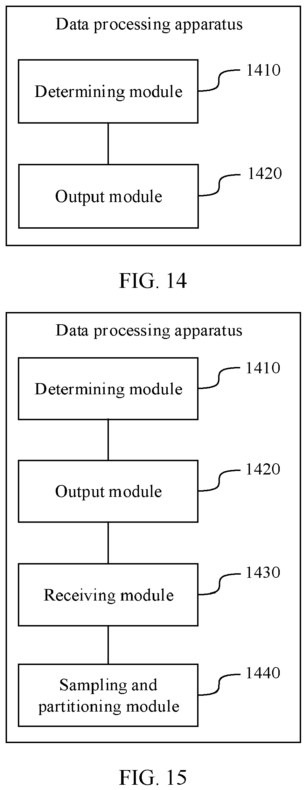

FIG. 14 is a diagram of a structure of a data processing apparatus according to an embodiment of this application. The apparatus may be implemented by using software, hardware, or a combination therefore, and become a part or an entirety of a server. The server provided in this embodiment of this application may implement the process described in FIG. 6 of embodiments of this application. The apparatus includes a determining module 1410 and an output module 1420.

The determining module 1410 is configured to:
determine, based on a query algorithm, a first query output corresponding to an input data set; and
determine, based on a second query output of an unsampled data record in the input data set and the query algorithm, a perturbation of each of a target quantity of sampled data records in the input data set to the first query output, to obtain sensitivity corresponding to the input data set, where the unsampled data record and the target quantity of data records constitute the input data set. The determining module 1410 may specifically implement the determining function in step 601, and other implicit steps.

The output module 1420 is configured to add noise to the first query output based on the sensitivity and output a noised first query output, and may specifically implement an output function in step 602, and other implicit steps.

In a possible implementation, as shown in FIG. 15, the apparatus further includes:
a receiving module 1430, configured to: before the first query output corresponding to the input data set is determined based on the query algorithm, receive a data query request, where the data query request includes the query algorithm; and
a sampling and partitioning module 1440, configured to randomly sample a target quantity of data records from the input data set corresponding to the data query request, to obtain the target quantity of data records and the unsampled data record in the input data set.

In a possible implementation, the determining module 1410 is configured to:
determine, based on the query algorithm, the second query output corresponding to the unsampled data record and a third query output corresponding to the target quantity of data records; and
determine, based on the second query output and the third query output, the first query output corresponding to the input data set.

In a possible implementation, the determining module 1410 is configured to:
determine, based on the query algorithm, the third query output corresponding to the target quantity of data records;
determine, based on the query algorithm, a query output provided after each of the target quantity of data records is deleted;
determine the perturbation of each of the target quantity of sampled data records to the first query output based on the second query output of the unsampled data record, the third query output, and the query output provided after each of the target quantity of data records is deleted; and
determine, as the sensitivity corresponding to the input data set, the maximum perturbation among the perturbations of the target quantity of data records to the first query output.

In a possible implementation, the sampling and partitioning module 1440 is further configured to:
before the noise is added to the first query output based on the sensitivity and the noised first query output is output, split the input data set into at least two partitions based on partitions to which the data records belong; and
the determining module 1410 is further configured to:
   determine current query outputs of the at least two partitions based on the query algorithm; and
   determine a difference between the current query output and a historical query output of each of the at least two partitions.

In a possible implementation, the determining module 1410 is further configured to:
for a target partition of the at least two partitions, if a current query output of the target partition is the same as a historical query output of the target partition, delete at least one data record from the target partition, so that a current query output and the historical query output of each of the at least two partitions are different;
determine, based on the query algorithm, a query output provided after the at least one data record is deleted from the target partition; and
determine, based on the query output provided after the at least one data record is deleted from the target partition, a fourth query output of the input data set; and
the output module 1420 is further configured to: add noise to the fourth query output based on the sensitivity and output a noised fourth query output.

Division into the modules in embodiments of this application is an example, is merely division into logical functions, and may be other division during actual implementation. In addition, functional modules in embodiments of this application may be integrated into one processor, or each of the modules may exist alone physically, or two or more modules may be integrated into one module. The integrated module may be implemented in a form of hardware, or may be implemented in a form of a software functional module.

The foregoing embodiment may be entirely or partially implemented by using software, hardware, firmware, or any combination thereof. When implemented by using software, the foregoing embodiment may be entirely or partially implemented in a form of a computer program product. The computer program product includes one or more computer instructions. When the computer instructions are loaded to and executed on a server or a terminal, the processes or functions in embodiments of this application may be entirely or partially produced. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial optical cable, an optical fiber, or a digital subscriber line) or wireless (for example, infrared, radio, or microwave) manner. The computer-readable storage medium may be any usable medium accessible by a server or a terminal, or a data storage device, such as a server or a data center, integrating one or more usable media. The usable medium may be a magnetic medium (for example, a floppy disk, a hard disk drive, or a tape), an optical medium (for example, a digital video disk (Digital Video Disk, DVD)), a semiconductor medium (for example, a solid-state drive), or the like.

## Claims

1. A data processing method, wherein the method comprises:
determining, based on a query algorithm, a first query output corresponding to an input data set;
determining, based on a second query output of an unsampled data record in the input data set and the query algorithm, a perturbation of each of a target quantity of sampled data records in the input data set to the first query output, to obtain sensitivity corresponding to the input data set, wherein the unsampled data record and the target quantity of data records constitute the input data set; and
adding noise to the first query output based on the sensitivity and outputting a noised first query output.

2. The method according to claim 1, wherein before the determining, based on a query algorithm, a first query output corresponding to an input data set, the method further comprises:
receiving a data query request, wherein the data query request comprises the query algorithm; and
randomly sampling a target quantity of data records from the input data set corresponding to the data query request, to obtain the target quantity of sampled data records and the unsampled data record in the input data set.

3. The method according to claim 1 or 2, wherein the determining, based on a query algorithm, a first query output corresponding to an input data set comprises:
determining, based on the query algorithm, the second query output corresponding to the unsampled data record and a third query output corresponding to the target quantity of data records; and
determining, based on the second query output and the third query output, the first query output corresponding to the input data set.

4. The method according to any one of claims 1 to 3, wherein the determining, based on a second query output of an unsampled data record in the input data set and the query algorithm, a perturbation of each of a target quantity of sampled data records in the input data set to the first query output, to obtain sensitivity corresponding to the input data set comprises:
determining, based on the query algorithm, the third query output corresponding to the target quantity of data records;
determining, based on the query algorithm, a query output provided after each of the target quantity of data records is deleted;
determining the perturbation of each of the target quantity of sampled data records to the first query output based on the second query output of the unsampled data record, the third query output, and the query output provided after each of the target quantity of data records is deleted; and
determining, as the sensitivity corresponding to the input data set, the maximum perturbation among the perturbations of the target quantity of data records to the first query output.

5. The method according to any one of claims 1 to 4, wherein before the adding noise to the first query output based on the sensitivity and outputting a noised first query output, the method further comprises:
splitting the input data set into at least two partitions based on partitions to which the data records belong;
determining current query outputs of the at least two partitions based on the query algorithm; and
determining a difference between the current query output and a historical query output of each of the at least two partitions.

6. The method according to claim 5, wherein the method further comprises:
for a target partition of the at least two partitions, if a current query output of the target partition is the same as a historical query output of the target partition, deleting at least one data record from the target partition, so that a current query output and the historical query output of each of the at least two partitions are different;
determining, based on the query algorithm, a query output provided after the at least one data record is deleted from the target partition;
determining, based on the query output provided after the at least one data record is deleted from the target partition, a fourth query output of the input data set; and
adding noise to the fourth query output based on the sensitivity and outputting a noised fourth query output.

7. A data processing apparatus, wherein the apparatus comprises:
a determining module, configured to:
determine, based on a query algorithm, a first query output corresponding to an input data set, and
determine, based on a second query output of an unsampled data record in the input data set and the query algorithm, a perturbation of each of a target quantity of sampled data records in the input data set to the first query output, to obtain sensitivity corresponding to the input data set, wherein the unsampled data record and the target quantity of data records constitute the input data set; and
an output module, configured to add noise to the first query output based on the sensitivity and output a noised first query output.

8. The apparatus according to claim 7, wherein the apparatus further comprises:
a receiving module, configured to: before the first query output corresponding to the input data set is determined based on the query algorithm, receive a data query request, wherein the data query request comprises the query algorithm; and
a sampling and partitioning module, configured to randomly sample a target quantity of data records from the input data set corresponding to the data query request, to obtain the target quantity of data records and the unsampled data record in the input data set.

9. The apparatus according to claim 7 or 8, wherein the determining module is configured to:
determine, based on the query algorithm, the second query output corresponding to the unsampled data record and a third query output corresponding to the target quantity of data records; and
determine, based on the second query output and the third query output, the first query output corresponding to the input data set.

10. The apparatus according to any one of claims 7 to 9, wherein the determining module is configured to:
determine, based on the query algorithm, the third query output corresponding to the target quantity of data records;
determine, based on the query algorithm, a query output provided after each of the target quantity of data records is deleted;
determine the perturbation of each of the target quantity of sampled data records to the first query output based on the second query output of the unsampled data record, the third query output, and the query output provided after each of the target quantity of data records is deleted; and
determine, as the sensitivity corresponding to the input data set, the maximum perturbation among the perturbations of the target quantity of data records to the first query output.

11. The apparatus according to any one of claims 7 to 10, wherein the sampling and partitioning module is further configured to:
before the noise is added to the first query output based on the sensitivity and the noised first query output is output, split the input data set into at least two partitions based on partitions to which the data records belong; and
the determining module is further configured to:
determine current query outputs of the at least two partitions based on the query algorithm; and
determine a difference between the current query output and a historical query output of each of the at least two partitions.

12. The apparatus according to claim 11, wherein the determining module is further configured to:
for a target partition of the at least two partitions, if a current query output of the target partition is the same as a historical query output of the target partition, delete at least one data record from the target partition, so that a current query output and the historical query output of each of the at least two partitions are different,
determine, based on the query algorithm, a query output provided after the at least one data record is deleted from the target partition, and
determine, based on the query output provided after the at least one data record is deleted from the target partition, a fourth query output of the input data set; and
the output module is further configured to add noise to the fourth query output based on the sensitivity and output a noised fourth query output.

13. A computing device for data processing, wherein the computing device comprises a processor and a memory;
the memory stores computer instructions; and
the processor executes the computer instructions, to implement the method according to any one of claims 1 to 6.

14. A computer-readable storage medium, wherein the computer-readable storage medium stores computer instructions, and when the computer instructions in the computer-readable storage medium are executed by a computing device, the computing device is enabled to perform the method according to any one of claims 1 to 6, or the computing device is enabled to implement functions of the apparatus according to any one of claims 7 to 12.
